# EUROPÄISCHE PATENTSCHRIFT

(11) **EP 2 709 982 B1**
(45) Veröffentlichungstag und Bekanntmachung des Hinweises auf die Patenterteilung: **18.02.2015**
(21) Anmeldenummer: 12726738.3
(22) Anmeldetag: 20.05.2012
(51) Int. Cl.: C07C 407/00, C07C 409/22, C07C 409/34, C08F 4/34, C08F 4/38

(54) **ARBEITSMEDIZINISCH UNBEDENKLICHE PULVERFÖRMIGE PEROXIDZUBEREITUNGEN**
POWDERY PEROXIDE COMPOSITIONS ACCEPTABLE FOR OCCUPATIONAL HEALTH
COMPOSITIONS DE PEROXYDE PULVÉRULENTES ACCEPTABLES POUR LA MÉDECINE DU TRAVAIL

(30) Priorität: 20.05.2011 DE 102011102682
(43) Veröffentlichungstag der Anmeldung: 26.03.2014
(73) Patentinhaber: Pergan GmbH, 46395 Bocholt (DE)
(72) Erfinder: VON ZADOW, Edgar, 46342 Velen (DE); SONDERMANN, Martin, 46414 Rhede (DE); WORMLAND, Bernhard, 46395 Bocholt (DE)
(74) Vertreter: Lütjens, Henning
(86) Internationale Anmeldenummer: PCT/EP2012/002144
(87) Internationale Veröffentlichungsnummer: WO 2012/159726

(56) Entgegenhaltungen:
- DE-A1- 1 618 726
- US-A- 5 334 326

## Beschreibung

Die vorliegende Erfindung betrifft arbeitsmedizinisch unbedenkliche pulverförmige Peroxidzubereitungen, Verfahren zu deren Herstellung sowie die Verwendung der arbeitsmedizinisch unbedenklichen pulverförmigen Peroxidzubereitungen in der chemischen Synthese, speziell zur Härtung von Harzen.

Organische Peroxide wie beispielsweise Dibenzoylperoxid, 2,4-Dichlorbenzoylperoxid oder Cyclohexanonperoxid werden als Initiatoren zur Härtung von Harzen wegen ihrer stoßempfindlichen und explosiven Eigenschaften nur in phlegmatisierter Form eingesetzt.

DE 16 18 726 beschreibt Verfahren zur Herstellung von pulverförmigen Zubereitungen, die feinverteilte Peroxidpulver mit guter Fließfähigkeit liefern. Diese Pulver bestehen aus Gemischen von bei Raumtemperatur festen Weichmacher, deren Schmelz- bzw. Erweichungstemperatur unter der Zerfallstemperatur des Peroxids liegen. Bevorzugt werden Phenylbenzoat, Dicyclohexylphthalat und Triphenylphosphat.

Die in obiger Auslegeschrift genannten festen Phlegmatisierungsmittel sind jedoch mittlerweile aus arbeitsmedizinischer Sicht problematisch und/oder zeigen anwendungstechnische Nachteile aufgrund zum Teil für viele Anwendungen zu niedriger Schmelz- bzw. Erweichungspunkte. So ist für einen effektiven Einsatz in vielen technischen Reaktionen ein Schmelzpunkt bzw. Erweichungspunkt von mehr als 45 °C erforderlich.

So enthält das im Handel erhältliche Dibenzoylperoxid als festes Phlegmatisierungsmittel bzw. Weichmacher in der Regel Dicyclohexylphthalat. Dieser Phthalsäureester unterliegt jedoch mittlerweile wie die meisten übrigen o-Phthalate einer strengen Kennzeichnung und ist somit aus arbeitsmedizinischer Sicht mit gegebener Vorsicht zu handhaben.

US 5,334,326 offenbart die Verwendung von Alkylmonobenzoaten mit 8 bis 12 Kohlenstoffatomen in der Alkylgruppe zur Stabilisierung von Diaroylperoxiden.

Es bestand daher ein Bedarf an Peroxidzubereitungen in Pulverform, bestehend aus einem festen Phlegmatisierungsmittel, das gegenüber dem Peroxid inert und in vernetzbaren Harzmassen löslich ist sowie keiner Kennzeichnung gemäß dem GHS unterliegt.

Überraschend wurde nun gefunden, dass bestimmte Alkylbenzoate als Phlegmatisierungsmittel für gängige organische Peroxide hervorragend geeignet sind. Solche Alkylbenzoate weisen die medizinische Bedenklichkeit der oben beschriebenen herkömmlichen Weichmacher nicht auf und erfordern somit weniger Sicherheitsmaßnahmen bei der Herstellung und Anwendung. weiterhin sind diese Alkylbenzoate ohne weiteres marktgängig verfügbar. Weiterhin weisen die erfindungsgemäß zu verwendenden Alkylbenzoate Schmelz- bzw. Erweichungspunkte von mehr als 45 °C sowie gute Löslichkeiten in Säurechloriden und Polyesterharzen auf.

Ein erster Gegenstand der vorliegenden Erfindung sind daher pulverförmige Peroxidzubereitungen umfassend mindestens ein organisches Peroxid sowie mindestens ein Alkylbenzoat mit einem Schmelz- bzw. Erweichungspunkt von mehr als 45°C, dadurch gekennzeichnet, dass das Alkylbenzoat Glycerintribenzoat, Neopentylglykoldibenzoat, Pentaerythritoltetrabenzoat, 1,4-Cyclohexandimethanoldibenzoat oder eine Mischung aus Glycerintribenzoat und Neopentylglykoldibenzoat ist.

Geeignete organische Peroxide sind alle gängigen Peroxide, beispielsweise und bevorzugt Dibenzoylperoxid, 2,4-Dichlorbenzoylperoxid und Cyclohexanonperoxid. Zur Vermeidung von Mißverständnissen sei hier betont, dass das organische Peroxid in der erfindungsgemäßen Peroxidzubereitung noch über die intakte Peroxofunktion verfügt, um beispielsweise als Initiator für chemische Reaktionen dienen zu können. Besonders bevorzugte Alkylbenzoate sind Glycerintribenzoat, Neopentylglykoldibenzoat sowie Mischungen dieser beiden Alkylbenzoate.

Das Gewichtsverhältnis zwischen organischem Peroxid und Alkylbenzoat beträgt von 90:10 bis 10:90, bevorzugt von 70:30 bis 30:70 und besonders bevorzugt von 60:40 bis 40:60.

In einer besonderen Ausführungsform wird als Alkylbenzoat eine Mischung von Glycerintribenzoat und Neopentylglykoldibenzoat verwendet, wobei deren Gewichtsverhältnis zueinander derart variiert wird, dass ein gewünschter Schmelz- bzw. Erweichungspunkt eingestellt wird.

Die erfindungsgemäßen pulverförmigen Peroxidzubereitungen weisen hervorragende anwendungstechnische Eigenschaften für den Einsatz in der chemischen Synthese, speziell als Initiatoren zur Härtung von Harzen auf, ohne aus arbeitsmedizinischer Sicht problematisch zu sein. So verursachen die erfindungsgemäßen pulverformigen Peroxidzubereitungen weniger Aufwand in der Handhabung bei der Herstellung und Anwendung, da ein arbeitsmedizinisch unbedenklicher Abrieb von festem Benzoat entsteht. Dies bedingt beispielsweise weniger Aufwand für Absaugeinrichtungen. Die erfindungsgemäßen pulverförmigen Peroxidzubereitungen weisen weiterhin auch ohne Zusatz von Rieselhilfen eine gute Rieselfähigkeit auf.

Zur Herstellung der erfindungsgemäßen pulverförmigen Peroxidzubereitungen sind die herkömmlichen Verfahren für die Herstellung pulverförmiger Peroxidzubereitungen geeignet, wie sie beispielsweise in DE 16 18 726 oder DE 17 68 199 offenbart sind.

Ein weiterer Gegenstand der vorliegenden Erfindung ist daher ein Verfahren zur Herstellung der erfindungsgemäßen pulverförmigen Peroxidzubereitungen umfassend das Vermischen mindestens eines in einem Lösemittel gelösten Alkylbenzoats mit einer wässrigen Suspension eines organischen Peroxids, das Abtrennen des Peroxid-Alkylbenzoat-Gemischs vom Wasser und Lösemittel und das Trocknen des erhaltenen Peroxid-Alkylbenzoat-Gemischs.

Ein weiterer Gegenstand der vorliegenden Erfindung ist ein Verfahren zur Herstellung der erfindungsgemäßen pulverförmigen Peroxidzubereitungen umfassend das Lösen mindestens eines Alkylbenzoats in einem Vorläufer des herzustellenden Peroxids, beispielsweise einem Keton oder Säurechlorid, das Umsetzen der erhaltenen Lösung mit einer Mischung aus wässriger Wasserstoffperoxid- und Natriumhydroxidlösung und die anschließende Entfernung des Wassers, gegebenenfalls mit nachfolgender Trocknung.

Ein weiterer Gegenstand der vorliegenden Erfindung ist die Verwendung der erfindungsgemäßen pulverförmigen Peroxidzubereitungen als Initiator einer chemischen Reaktion. In einer bevorzugten Ausführungsform handelt es sich bei der chemischen Reaktion um eine Härtung von Harzen.

Beispiel 1. In einer Mischung aus 230,0 kg Wasser und 22,5 kg Natronlauge 45 %ig werden 23,0 kg Glycerintribenzoat fein dispergiert und auf 5 °C abgekühlt. Zu dieser Suspension werden unter intensivem Rühren 1,75 kg Netzmittel (Na-Alkylbenzolsulfonat), 1,5 kg Isohexan sowie 5,3 kg Wasserstoffperoxid 60 %ig zugegeben. Anschließend werden innerhalb von 10 Minuten 29,5 kg Benzoylchlorid bei 18 - 20 °C zugetropft und danach 20 Minuten bei 20 °C weitergerührt. Nach Abtrennung, Waschen und Trocknen wurden 47 kg eines feinen Granulates mit einem Gehalt von 49,5 % Benzoylperoxid erhalten.

Beispiel 2. In eine Mischung aus 230 kg Wasser, 22,5 kg Natronlauge 40 %ig, 5,3 kg Wasserstoffperoxid und 1,75 kg Netzmittel (Na-Alkylbenzolsulfonat) wird unter Rühren und Kühlen auf ca. 18 - 20 °C innerhalb von 10 Minuten eine Mischung von 23,0 kg Glycerintribenzoat in 29,3 kg Benzoylchlorid, bei 30 °C gelöst, hinzugegeben. Nach weiterem Rühren bei 20 °C wird das gebildete Feingranulat von der Mutterlauge abgetrennt, gewaschen und getrocknet. Es wurden 46,0 kg Benzoylperoxid 50,5 % mit Glycerintribenzoat phlegmatisiert erhalten.

## Patentansprüche

1. Pulverförmige Peroxidzubereitung umfassend mindestens ein organisches Peroxid sowie mindestens ein Alkylbenzoat mit einem Schmelz- bzw. Erweichungspunkt von mehr als 45 °C **dadurch gekennzeichnet, dass** das Alkylbenzoat Glycerintribenzoat, Neopentylglykoldibenzoat, Pentaerythritoltetrabenzoat, 1,4-Cyclohexandimethanoldibenzoat oder eine Mischung aus Glycerintribenzoat und Neopentylglykoldibenzoat ist.

2. Pulverförmige Peroxidzubereitung gemäß Anspruch 1, **dadurch gekennzeichnet, dass** das organische Peroxid mindestens eines ausgewählt aus der Gruppe umfassend Dibenzoylperoxid, 2,4-Dichlorbenzoylperoxid und Cyclohexanonperoxid ist.

3. Pulverförmige Peroxidzubereitung gemäß einem der vorstehenden Ansprüche, **dadurch gekennzeichnet, dass** das Alkylbenzoat Glycerintribenzoat, Neopentylglykoldibenzoat oder eine Mischung daraus ist.

4. Pulverförmige Peroxidzubereitung gemäß einem der vorstehenden Ansprüche, **dadurch gekennzeichnet, dass** das Gewichtsverhältnis zwischen organischem Peroxid und Alkylbenzoat von 90:10 bis 10:90 beträgt.

5. Verfahren zur Herstellung einer pulverförmigen Peroxidzubereitung gemäß einem der Ansprüche 1 bis 4 umfassend das Vermischen mindestens eines in einem Lösemittel gelösten Alkylbenzoats ausgewählt aus der Gruppe bestehend aus Glycerintribenzoat, Neopentylglykoldibenzoat, Pentaerythritoltetrabenzoat und 1,4- Cyclohexandimethanoldibenzoat mit einer wässrigen Suspension eines organischen Peroxids, das Abtrennen des Peroxid-Alkylbenzoat-Gemischs vom Wasser und Lösemittel und das Trocknen des erhaltenen Peroxid-Alkylbenzoat-Gemischs.

6. Verfahren zur Herstellung einer pulverförmigen Peroxidzubereitung gemäß einem der Ansprüche 1 bis 4 umfassend das Lösen mindestens eines Alkylbenzoats ausgewählt aus der Gruppe bestehend aus Glycerintribenzoat, Neopentylglykoldibenzoat, Pentaerythritoltetrabenzoat und 1,4-Cyclohexandimethanoldibenzoat in einem Vorläufer des herzustellenden Peroxids, das Umsetzen der erhaltenen Lösung mit einer Mischung aus wässriger Wasserstoffperoxid- und Natriumhydroxidlösung und die anschließende Entfernung des Wassers, gegebenenfalls mit nachfolgender Trocknung.

7. Verfahren gemäß Anspruch 6, **dadurch gekennzeichnet, dass** der Vorläufer des herzustellenden Peroxids ein Keton oder Säurechlorid ist.

8. Verwendung einer pulverförmigen Peroxidzubereitung gemäß einem der Ansprüche 1 bis 4 als Initiator einer chemischen Reaktion.

9. Verwendung gemäß Anspruch 8, **dadurch gekennzeichnet, dass** die chemische Reaktion die Härtung eines Harzes ist.

## Claims

1. Pulverulent peroxide preparation comprising at least one organic peroxide and at least one alkyl benzoate with a melting or softening point above 45°C, **characterized in that** the alkyl benzoate is glycerol tribenzoate, neopentyl glycol dibenzoate, pentaerythritol tetrabenzoate, 1,4-cyclohexanedimethanol dibenzoate or a mixture of glycerol tribenzoate and neopently glycol dibenzoate.

2. Pulverulent peroxide preparation according to Claim 1, **characterized in that** the organic peroxide is at least one selected from the group comprising dibenzoyl peroxide, 2,4-dichlorobenzoyl peroxide and cyclohexanone peroxide.

3. Pulverulent peroxide preparation according to any of the preceding claims, **characterized in that** the alkyl benzoate is glycerol tribenzoate, neopentyl glycol dibenzoate or a mixture thereof.

4. Pulverulent peroxide preparation according to any of the preceding claims, **characterized in that** the ratio by weight of organic peroxide to alkyl benzoate is from 90:10 to 10:90.

5. Process for the production of a pulverulent peroxide preparation according to any of Claims 1 to 4 comprising the mixing of, dissolved in a solvent, at least one alkyl benzoate selected from the group consisting of glycerol tribenzoate, neopentyl glycol dibenzoate, pentaerythritol tetrabenzoate and 1,4-cyclohyexanedimethanol dibenzoate with an aqueous suspension of an organic peroxide, the isolation of the peroxide-alkyl-benzoate mixture from the water and solvent and the drying of the resultant peroxide-alkylbenzoate mixture.

6. Process for the production of a pulverulent peroxide preparation according to any of Claims 1 to 4 comprising the dissolution of at least one alkyl benzoate selected from the group consisting of glycerol tribenzoate, neopentyl glycol dibenzoate, pentaerythritol tetrabenzoate and 1,4-cyclohexanedimethanol dibenzoate in a precursor of the peroxide to be produced, the reaction of the resultant solution with a mixture of aqueous hydrogen peroxide solution and aqueous sodium hydroxide solution and then the removal of water, optionally with subsequent drying.

7. Process according to Claim 6, **characterized in that** the precursor of the peroxide to be produced is a ketone or acyl chloride.

8. Use of a pulverulent peroxide preparation according to any of Claims 1 to 4 as initiator of a chemical reaction.

9. Use according to Claim 8, **characterized in that** the chemical reaction is the curing of a resin.

## Revendications

1. Composition de peroxyde sous forme de poudre comprenant au moins un peroxyde organique ainsi qu'au moins un benzoate d'alkyle présentant un point de fusion ou de ramollissement supérieur à 45°C, **caractérisée en ce que** le benzoate d'alkyle est le tribenzoate de glycérol, le dibenzoate de néopentylglycol, le tétrabenzoate de pentaérythritol, le dibenzoate de 1,4-cyclohexanediméthanol ou un mélange de tribenzoate de glycérol et de dibenzoate de néopentylglycol.

2. Composition de peroxyde sous forme de poudre selon la revendication 1, **caractérisée en ce que** le peroxyde organique est au moins un peroxyde choisi dans le groupe comprenant le peroxyde de dibenzoyle, de peroxyde de 2,4-dichlorobenzoyle et le peroxyde de cyclohexanone.

3. Composition de peroxyde sous forme de poudre selon l'une quelconque des revendications précédentes, **caractérisée en ce que** le benzoate d'alkyle est le tribenzoate de glycérol, le dibenzoate de néopentylglycol ou un mélange de ceux-ci.

4. Composition de peroxyde sous forme de poudre selon l'une quelconque des revendications précédentes, **caractérisée en ce que** le rapport pondéral entre le peroxyde organique et le benzoate d'alkyle est de 90:10 à 10:90.

5. Procédé pour la préparation d'une composition de peroxyde sous forme de poudre selon l'une quelconque des revendications 1 à 4, comprenant le mélange d'au moins un benzoate d'alkyle dissous dans un solvant choisi dans le groupe constitué par le tribenzoate de glycérol, le dibenzoate de néopentylglycol, le tétrabenzoate de pentaérythritol et le dibenzoate de 1,4-cyclohexanediméthanol avec une suspension aqueuse d'un peroxyde organique, la séparation du mélange peroxyde-benzoate d'alkyle de l'eau et du solvant et le séchage du mélange peroxyde-benzoate d'alkyle obtenu.

6. Procédé pour la préparation d'une composition de peroxyde sous forme de poudre selon l'une quelconque des revendications 1 à 4, comprenant la dissolution d'au moins un benzoate d'alkyle choisi dans le groupe constitué par le tribenzoate de glycérol, le dibenzoate de néopentylglycol, le tétrabenzoate de pentaérythritol et le dibenzoate de 1,4-cyclohexanediméthanol dans un précurseur du peroxyde à préparer, la transformation de la solution obtenue avec un mélange d'une solution aqueuse de peroxyde d'hydrogène et d'une solution aqueuse d'hydroxyde de sodium et l'élimination consécutive de l'eau, le cas échéant suivie d'un séchage.

7. Procédé selon la revendication 6, **caractérisé en ce que** le précurseur du peroxyde à préparer est une cétone ou un chlorure d'acide.

8. Utilisation d'une composition de peroxyde sous forme de poudre selon l'une quelconque des revendications 1 à 4 comme initiateur d'une réaction chimique.

9. Utilisation selon la revendication 8, **caractérisée en ce que** la réaction chimique est le durcissement d'une résine.
